# EUROPEAN PATENT APPLICATION

(11) **EP 4 302 683 A1**
(43) Date of publication of application: **10.01.2024**
(21) Application number: 22784795.1
(22) Date of filing: 16.03.2022
(51) Int. Cl.: A61B 5/00, A61B 5/024, A61B 5/256, A61B 5/28, A61B 5/1455, A61B 5/021, A61B 5/145

(54) **WEARABLE ELECTRONIC DEVICE**

(30) Priority: 06.04.2021 KR 20210044531
(71) Applicant: Samsung Electronics Co., Ltd., Suwon-si, Gyeonggi-do 16677 (KR)
(72) Inventor: JEONG, Injo, Suwon-si, Gyeonggi-do 16677 (KR); PRUSHINSKIY, Valeriy, Suwon-si, Gyeonggi-do 16677 (KR); HYUN, Suk, Suwon-si, Gyeonggi-do 16677 (KR); YOO, Hyunguk, Suwon-si, Gyeonggi-do 16677 (KR); KIM, Younghyun, Suwon-si, Gyeonggi-do 16677 (KR); LEE, Suho, Suwon-si, Gyeonggi-do 16677 (KR)
(74) Representative: Appleyard Lees IP LLP
(86) International application number: PCT/KR2022/003650
(87) International publication number: WO 2022/215885

(57) **Abstract**

A wearable electronic device according to an embodiment comprises: a housing; and a first sensor which detects first biometric information on the basis of light diffracted or reflected by an external object, wherein the first sensor comprises: a light source located in the housing; a plurality of photodetectors which are located in the housing and detect the light; and a light guide member extending from an area adjacent to the light source to a peripheral area of the plurality of photodetectors, wherein light incident thereinto travels while being totally reflected, wherein the light guide member comprises: a light incident portion which is adjacent to the light source and to which light emitted from the light source is incident; an adjacent portion located in the peripheral area of the plurality of photodetectors; and a plurality of light output portions which are located on one surface of the adjacent portion and from which the incident light is emitted.

## Description

### [Technical Field]

Various embodiments disclosed in the disclosure relate to a wearable electronic device.

### [Background Art]

In recent years, as wearable electronic devices have been widely distributed, demands on wearable electronic devices equipped with more various functions have been increased.

In particular, because the wearable electronic devices may be used while the users carrying them conveniently and easily, methods for detecting biometric information have been studied by using the wearable electronic devices. A health care service for a user may be provided by detecting biometric information by using the wearable electronic devices. To provide a more precise health care service, methods for detecting biometric information have been studied.

### [Disclosure]

### [Technical Problem]

Various embodiments may provide a wearable electronic device, in which an area, in which light is emitted, is disposed to be adjacent to an area, in which the light is detected, such that a preciseness of the detected biometric information is enhanced.

Various embodiments may provide a wearable electronic device, in which an area occupied by a sensor is reduced such that a degree of freedom of design of other components of the electronic device is enhanced.

### [Technical Solution]

A wearable electronic device according to an embodiment may include a housing, and a first sensor that detects first biometric information based on light diffracted or reflected by an external object, the first sensor may include a light source located inside the housing, a plurality of light detectors located inside the housing and that detects the light, and a light guide member extending from an area that is adjacent to the light source to a peripheral area of the plurality of light detectors, and in which the light input to an interior thereof travels while being totally reflected, and the light guide member may include a light input part being adjacent to the light source and to which the light emitting from the light source is input, an adjacent part located at the peripheral area of the plurality of light detectors, and a plurality of light output parts located on one surface of the adjacent part and from which the input light is emitted.

A wearable electronic device according to an embodiment may include a housing, a first sensor that detects first biometric sensor, and a second sensor including a plurality of electrodes located on a rear surface of the housing and that detects second biometric information, the first sensor may include a light source located on an outside of a central area of a rear surface of the housing, inside the housing, and that overlaps at least any one of the plurality of electrodes, a plurality of light detectors located inside the housing and that detects light, and a light guide member extending from an area that is adjacent to the light source to a peripheral area of the plurality of light detectors, and through which the light input to an interior thereof travels while being totally reflected, the light guide member may include a light input part, to which the light emitted from the light source is input, an adjacent part located in the peripheral area of the plurality of light detectors, and a plurality of light output parts located in the peripheral area of the plurality of light detectors and from which the input light is emitted, and the light output part and the light detector may not overlap the plurality of electrodes.

### [Advantageous Effects]

According to various embodiments, in the wearable electronic device, an area, in which light is emitted, is disposed to be adjacent to an area, in which the light is detected, such that a preciseness of the detected biometric information is enhanced.

According to various embodiments, in the wearable electronic device, an area occupied by a sensor is reduced such that a degree of freedom of design of other components of the electronic device is enhanced.

### [Description of Drawings]

FIG. 1 is a block diagram illustrating an example electronic device in a network environment according to various embodiments.
FIG. 2 is a front perspective view of an electronic device according to an embodiment.
FIG. 3 is a rear perspective view of an electronic device according to an embodiment.
FIG. 4 is a rear view illustrating an electronic device according to an embodiment.
FIG. 5 is a view of a first sensor included in an electronic device according to an embodiment.
FIG. 6 is a cross-sectional view illustrating some configurations included in an electronic device according to an embodiment.
FIG. 7 is a cross-sectional view illustrating an electronic device according to an embodiment.
FIG. 8 is a view illustrating some configurations of an electronic device according to an embodiment.
FIG. 9 is a rear view of an electronic device according to an embodiment.
FIG. 10 is a rear view of an electronic device according to an embodiment.
FIG. 11 is a rear view of an electronic device according to an embodiment.
FIG. 12 is a rear view of an electronic device according to an embodiment.
FIG. 13 is a view illustrating some configurations of an electronic device according to an embodiment.
FIG. 14 is a rear view of an electronic device according to an embodiment.
FIG. 15 is a view illustrating some configurations of an electronic device according to an embodiment.
FIG. 16 is a cross-sectional view illustrating some configurations of an electronic device according to an embodiment.
FIG. 17 is a view illustrating some configurations included in an electronic device according to an embodiment.
FIG. 18 is a view illustrating some configurations included in an electronic device according to an embodiment.

In relation to a description of the drawings, the same or similar components may be denoted by the same or similar reference numerals.

### [Mode for Invention]

Hereinafter, various embodiments of the disclosure will be described with reference to the accompanying drawings. However, it should be understood that the disclosure is not limited to specific embodiments and includes various modifications, equivalents, and/or alternatives of the embodiments of the disclosure.

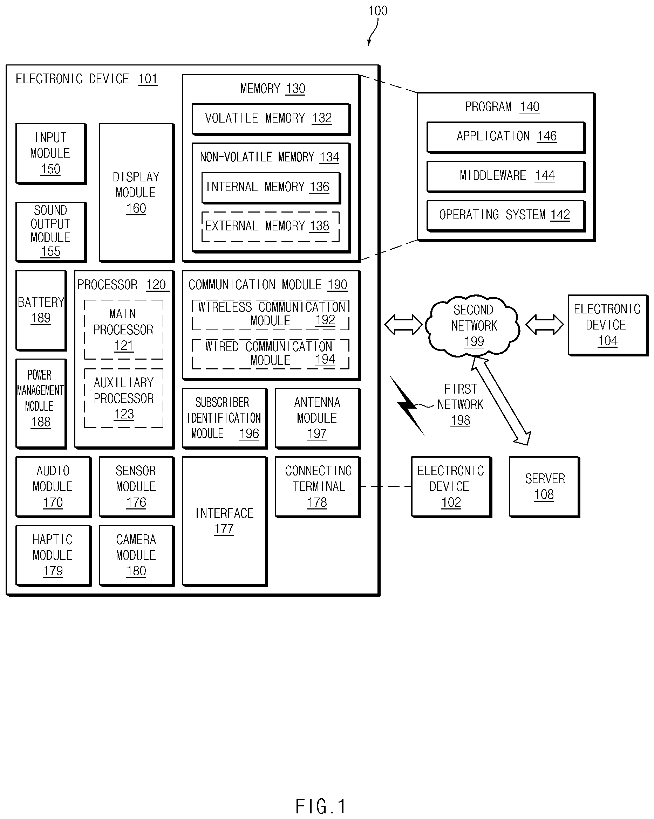

FIG. 1 is a block diagram illustrating an electronic device 101 in a network environment 100 according to various embodiments. Referring to FIG. 1, an electronic device 101 in a network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the millimeter (mm) Wave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-ofthings (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

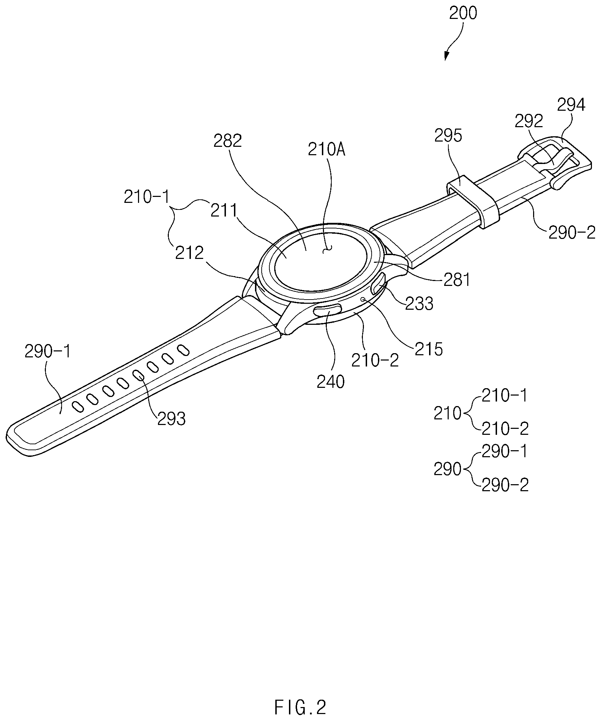

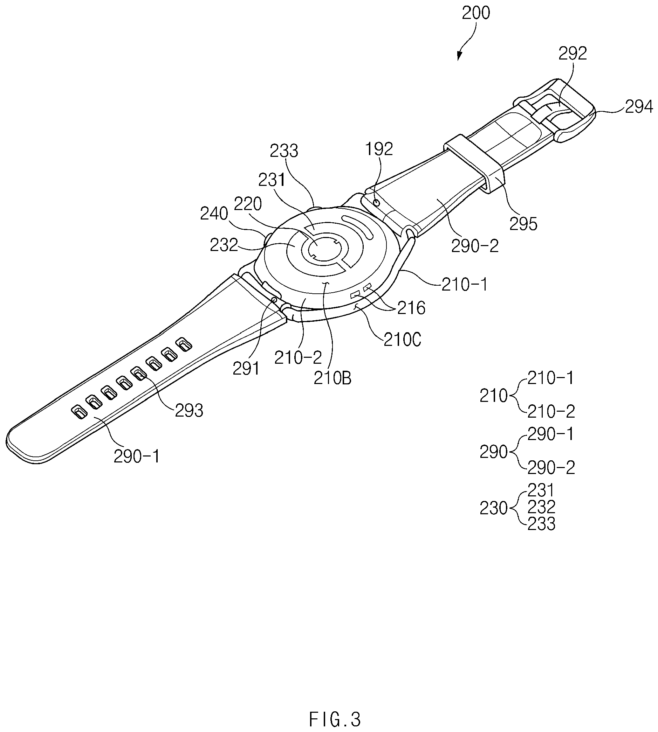

FIG. 2 is a front perspective view of an electronic device according to an embodiment. FIG. 3 is a rear perspective view of an electronic device according to an embodiment.

Referring to FIGS. 2 and 3, an electronic device 200 according to an embodiment may include a housing 210, key input devices 240 and 281, audio modules 215 and 216, sensor modules 220 and 230, and a fastening member 290.

The electronic device 200 according to an embodiment may be a wearable electronic device. For example, the electronic device 200 may be an electronic device (e.g., a smart watch) of a watch type, which may be mounted on a portion (e.g., a wrist) of the body of the user. However, the electronic device is not limited to the illustrated embodiment.

In an embodiment, the housing 210 may define at least a portion of an external appearance of the electronic device 200. The housing 210 may include a front case 210-1 and a rear case 210-2. The front case 210-1 and the rear case 210-2 may be coupled to each other. For example, the housing 210 may provide an interior space, in which other components (e.g., a bracket, a circuit board, a display, and/or a battery) of the electronic device 200 may be accommodated, through a coupling structure of the front case 210-1 and the rear case 210-2.

In an embodiment, the housing 210 may include a front surface 210A, a rear surface 210B that faces an opposite direction to the front surface 210A, and a side surface 210C that surround a space between the front surface 210A and the rear surface 210B. For example, the front case 210-1 may define at least a portion of the front surface 210A and the side surface 210C of the housing 210, and the rear case 210-2 may define at least a portion of the rear surface 210B of the housing 210. The front case 210-1 may include a front plate 211 that defines at least a portion of the front surface 210A, and a side frame 212 (e.g., a side bezel or a side member) that defines at least a portion of the side surface 210C. According to various embodiments, the housing 210 may refer to a structure that defines a portion of the front surface 210A, the rear surface 210B, and the side surface 210C.

In an embodiment, the front surface 210A and the side surface 210C may be included in the front case 210-1, and the rear surface 210B may be included in the rear case 210-2. For example, at least a portion of the front surface 210A may be defined by the substantially transparent front plate 211. The front plate 211 may be formed of a glass plate including various coating layers, or a polymer plate. The rear surface 210B may be formed of the substantially opaque rear case 210-2. The rear case 210-2 may be formed of coated or colored glass, ceramics, a polymer metal (e.g., aluminum, stainless steel (STS), or magnesium), or a combination of at least two of the materials. The side surface 210C may be defined by the side frame 212 (e.g., the side bezel or the side member) coupled to the front plate 211 and the rear case 210-2. The side frame 212 may include a metal and/or a polymer. According to various embodiments, the side frame 212 may be integrally formed with the rear case 210-2. For example, the rear case 210-2 and the side frame 212 may be integrally formed, and may include the same material (e.g., a metallic material such as aluminum).

The electronic device 200 according to an embodiment may include a display 282 that is disposed in an interior of the housing 210 and is visually exposed to an outside of the electronic device 200. For example, at least a portion of the display 282 may be visually exposed to the front surface 210A of the housing 210 through the substantially transparent front plate 211. The display 282 may be formed in a shape corresponding to a shape of the front plate 211. For example, the display 282 may have various shapes, such as a circular shape, an elliptical shape, or a polygonal shape. The display 282 may be coupled to a touch detecting circuit, a pressure sensor that may measure an intensity (pressure) of a touch, and/or a fingerprint sensor, but may be disposed to be adjacent thereto.

In an embodiment, the audio modules 215 and 216 may include a microphone hole 215 and a speaker hole 216. For example, a microphone for acquiring an external sound may be disposed in an interior of the microphone hole 215. According to various embodiments, a plurality of microphones may be disposed in the electronic device 200 to detect sounds of various directions. A speaker for outputting a sound to an outside may be disposed in an interior of the speaker hole 216, and may be used as an external speaker and a communication receiver. According to various embodiments, the speaker hole 216 and the microphone hole 215 may be implemented by one hole. Alternatively, the electronic device 200 may include a speaker (e.g., a piezo speaker) with no speaker hole 216.

In an embodiment, the sensor modules 220 and 230 may detect biometric information, and may generate an electrical signal or a data value corresponding to the biometric information. For example, the sensor modules 220 and 230 may include a first sensor 220 (e.g., a heat rate measuring (HRM) sensor or a temperature sensor) disposed on the rear surface 210B of the housing 210, and a second sensor 230 including at least one electrode 231, 232, and 233. Although not illustrated, according to various embodiments, the electronic device 200 may further include at least one of a gesture sensor, a gyro sensor, an atmospheric pressure sensor, an acceleration sensor, a grip sensor, a color sensor, an infrared ray (IR) sensor, a temperature sensor, a humidity sensor, or an illumination intensity sensor.

In an embodiment, the second sensor 230 may include the at least one electrode 231, 232, and 233 that is exposed to the rear surface 210B of the housing 210. The at least one electrode 231, 232, and 233 may include the first electrode 231, the second electrode 232, and the third electrode 233. The second sensor 230 may be configured to acquire an electrical signal from a portion of the body of the user through the at least one electrode 231, 232, and 233 and detect biometric information (e.g., electrocardiogram (ECG)) of the user based on the acquired electrical signal.

In an embodiment, the key input devices 240 and 281 may include the wheel member 281 (e.g., a wheel key or a rotary bezel) that is disposed on the front surface 210A of the housing 210 and may be rotated in at least one direction, and/or the button member 240 (e.g., a side key) that is disposed on the side surface 210C of the housing 210.

In an embodiment, the wheel member 281 may have a shape (e.g., a circular frame) corresponding to a shape of the front plate 211. For example, the wheel member 281 may be rotated through manipulation by the user to receive the user input for implementing various functions of the electronic device 200. The button member 240 may be rotated and/or pressed through manipulation by the user to receive the user input for implementing various functions of the electronic device 200. The electronic device 200 may not include some or all of the above-described key input devices 240 and 281, and the key input device(s) 240 and 281 that is not included may be implemented in another form, for example, a form of a soft key on the display 282.

In an embodiment, the fastening member 290 may detachably mount the electronic device 200 on a portion (e.g., a wrist) of the body of the user. For example, the fastening member 290 may be connected to at least a portion of the housing 210, and may be detachably fastened while surrounding a portion of the body of the user. For example, the fastening member 290 may include a first fastening member 290-1 and a second fastening member 290-2 that are coupled to opposite sides of the housing 210, respectively. The first fastening member 290-1 and the second fastening member 290-2 may be connected to or separated from each other.

In an embodiment, the fastening member 290 may be formed in a band or strip shape to surround a portion of the body of the user. For example, the fastening member 290 may be formed integrally and such that a plurality of unit links are moved with respect to each other through fabrics, leather, rubber, urethane, metal, ceramics, or a combination of at least two of the materials.

In an embodiment, the fastening member 290 may be detachably connected to the housing 210. For example, the fastening member 290 may be detachably connected to at least a partial area of the housing 210 by using locking members 291 and 292. The electronic device 200 according to various embodiments may be provided with various kinds of fastening members 290, and the fastening member 290 may be replaced according to a taste and/or a preference of the user.

In an embodiment, the fastening member 290 may include a fixing member 292, a fixing member fastening hole 293, a band guide member 294, and/or a band fixing ring 295. For example, the fixing member 292 may be configured to fix the housing 210 and the fastening member 290 to a portion (e.g., a wrist or a wrinkle) of the body of the user. The fixing member fastening hole 293 may fix the housing 210 and the fastening member 290 to a portion of the body of the user in correspondence to the fixing member 292. The band guide member 294 may be configured to restrict a movement range of the fixing member 292 when the fixing member 292 is fastened to the fixing member fastening hole 293 such that the fastening member 290 is adhered and fastened to the portion of the body of the user. The band fixing ring 295 may restrict a movement range of the fastening member 290 while the fixing member 292 and the fixing member fastening hole 293 are fastened to each other.

According to various embodiments, the electronic device 200 may exclude at least one of the components illustrated in FIGS. 2 and 3, or additionally include another component. For example, the electronic device 200 may further include a connector hole (not illustrated). The connector hole (not illustrated) may accommodate a connector (e.g., a USB connector) for transmitting and/or receiving electric power and/or data to and from an external electronic device, or may accommodate a connector (e.g., an earphone connector) for transmitting and/or receiving an audio signal to and from the external electronic device.

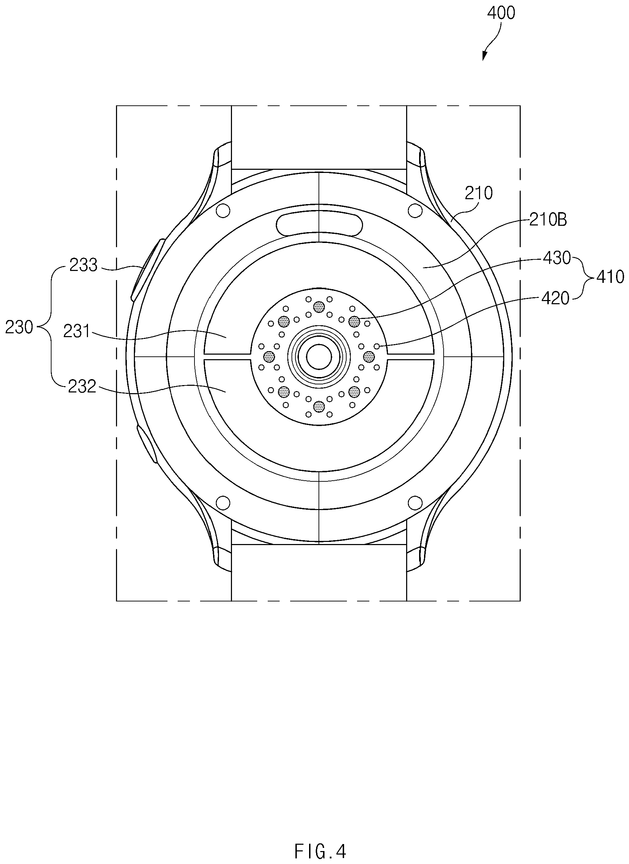

Referring to FIG. 4, an electronic device 400 according to an embodiment will be described. FIG. 4 is a rear view illustrating the electronic device 400 according to an embodiment. The electronic device 400 according to an embodiment may include the housing 210, a first sensor 410, and the second sensor 230.

The first sensor 410 may include a plurality of light output parts 420 that emits light to an outside of the electronic device 400, and a plurality of light detectors 430 that detects light. The first sensor 410 may detect first biometric information (e.g., a heart rate, an oxygen saturation, a blood pressure, and/or blood glucose) based on the light that is emitted from the light output part 420, diffracted or reflected by an external object (e.g., a wrist or a blood vessel of the user), and is input to the light detector 430. For example, the first sensor 410 may be a photoplethysmogram (PPG) sensor.

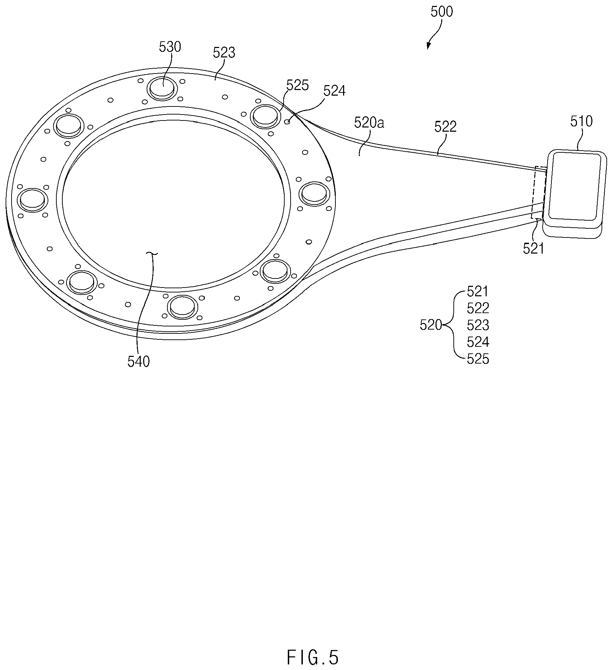

The plurality of light output parts 420 may emit the light emitted from a light source (e.g., a light source 510 of FIG. 5) located inside the housing 210 to an outside of the electronic device 400. The plurality of light output parts 420 may be located in at least one area of the rear surface 210b of the housing 210. The plurality of light output parts 420 may be disposed to be adjacent to at least one of the plurality of light detectors 430. For example, the plurality of light output parts 420 may be located in a peripheral area of any one of the plurality of light detectors 430.

The plurality of light detectors 430 may detect light and may detect an intensity of the detected light. For example, the plurality of light detectors 430 may output a current signal of a magnitude corresponding to a detected amount of light. The plurality of light detectors 430 may be located in at least one area of the rear surface 210b of the housing 210. For example, the plurality of light detectors 430 may be disposed to surround a central area of the rear surface 210b of the housing 210. The plurality of light detectors 430 may be surrounded by the first electrode 231 and the second electrode 232, on the rear surface 210b of the housing 210.

According to an embodiment, the first sensor 410 may further include an optical signal processing circuit (not illustrated) that is electrically connected to the light source and the plurality of light detectors 430. The optical signal processing circuit may acquire and process a current signal through the plurality of light detectors 430. The optical signal processing circuit may detect the biometric information of the user by using the electrical signal acquired through the plurality of light detectors 430.

The second sensor 230 may include the first electrode 231, the second electrode 232, and the third electrode 233. The first electrode 231 and the second electrode 232 may be located in at least one area of the rear surface 210b of the housing 210. The third electrode 233 may be located in one area of the side surface of the housing 210. The first electrode 231, the second electrode 232, and the third electrode 233 may acquire an electrical signal while contacting an external object (e.g., skin of the user). For example, the first electrode 231 and the third electrode 233 may correspond to an INP electrode and an INN electrode that acquire electrical signals at different locations of the external object, respectively. The second electrode 232 may correspond to an RLD electrode used for reduction of in-phase component noise and biological biasing. For example, the electronic device 400 according to an embodiment may offset in-phase component noise by outputting the same signal as the in-phase component noise that is input to the first electrode 231 and the third electrode 233 through the second electrode 232, and a biometric information detection performance of the second sensor 230 may be enhanced. The second sensor 230 may detect second biometric information (e.g., an electrocardiogram (ECG), a bioelectrical impedance analysis (BIA), and/or an electrodermal activity (EDA)) of the user based on an electrical signal acquired by using the first electrode 231, the second electrode 232, and the third electrode 233.

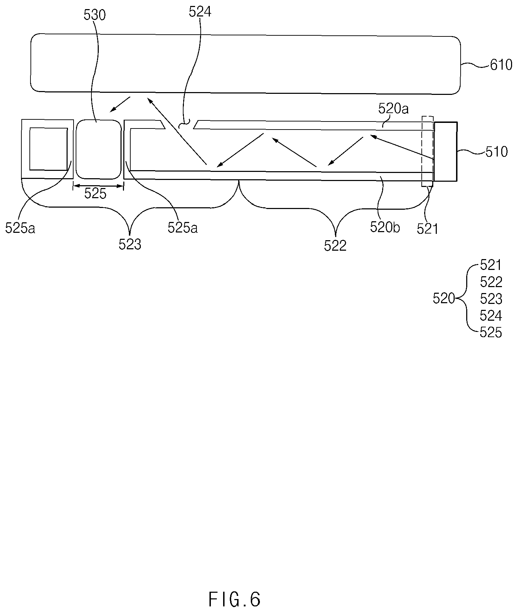

Hereinafter, referring to FIGS. 5 and 6, a first sensor 500 (e.g., the first sensor 410 of FIG. 4) included in the electronic device according to an embodiment will be described. FIG. 5 is a view of the first sensor 500 included in the electronic device according to an embodiment. FIG. 6 is a cross-sectional view illustrating some configurations included in the electronic device according to an embodiment.

According to an embodiment, the first sensor 500 may include the light source 510, a light guide member 520, and a plurality of light detectors 530.

The light source 510 may include at least one light emitting element (e.g., a light emitting diode (LED)) for irradiating light of a wavelength of a specific range. For example, the light emitting element may be configured to emit lights of different wavelengths. As another example, at least a portion of the light emitting element may be configured to emit light of the same wavelength. As another example, each of the light emitting elements may emit light at the same time point or may emit light based on a specific pattern. The light source 510 may be located on an outside of a central area 540 of the rear surface of the housing (e.g., the housing 210 of FIG. 3).

The light guide member 520 may guide the light emitted from the light source 510 such that the light is emitted to an outside of the electronic device through a light output part 524 located in a peripheral area of the light detector 530. The light input to the light guide member 520 may travel while being totally reflected in the light guide member 520. For example, the light guide member 520 may include poly methyl methacrylate (PMMA) or polyethylene (PE). According to an embodiment, the light guide member 520 may further include at least one of a black coating layer and a metal layer for inducing total reflection of the light. The light guide member 520 may include a light input part 521, a connection part 522, an adjacent part 523, a plurality of light output parts 524, and as plurality of through-holes 525. Furthermore, the light guide member 520 may include a first surface 520a that faces a rearward direction of the electronic device and a second surface 520b that faces the first surface 520a.

The light input part 521 may contact the light source 510. The light input part 521 may include a light transmitting material, or may include a space, by which the light may be input to the light guide member 520.

The connection part 522 may extend from the light input part 521 to the adjacent part 523, and may connect the light input part 521 and the adjacent part 523. According to an embodiment, the connection part 522 may be omitted.

The adjacent part 523 may be located in an area that is adjacent to the plurality of light detectors 530. The adjacent part 523 may extend along a circumference of the central area 540. For example, the central area 540 may be circular, and the adjacent part 523 may have a ring shape. At least one configuration included in the electronic device may be disposed in the central area 540.

The plurality of light output parts 524 may be located in a peripheral area of at least any one of the plurality of light detectors 530, in the adjacent part 523. The plurality of light output parts 524 may correspond to a hole that passes the first surface 523a, in the adjacent part 523. The plurality of light output parts 524 may not overlap the light source 510. According to an embodiment, the light emitted from the light source 510 may be delivered through the light guide member 520 and may be emitted from the plurality of light output parts 524 disposed to be adjacent to the light detector 530, to an outside of the electronic device.

A through-hole 525 may pass through a second surface 523b from the first surface 523a of the light guide member 520, in the adjacent part 523. The adjacent part 523 may include a side wall 525a that defines the through-hole 525. The adjacent part 523 may include the side wall 525a such that the light is prevented from being leaked from the through-hole 525. The plurality of light output parts 524 may be disposed in a peripheral area of the through-hole 525.

The plurality of light detectors 530 may be adjacent to the adjacent part 523 and the plurality of light output parts 524 of the light guide member 520. The plurality of light detectors 530 may be adjacent to at least one surface of the adjacent part 523. For example, the plurality of light detectors 530 may be located in the through-hole 525 of the light guide member 520. The plurality of light detectors 530 may be surrounded by the side wall 525a. A width of the through-hole 525 may be equal to or larger than a width of the light detector 530 located in the through-hole 525. For example, an empty space may be located between the side wall 525a of the through-hole 525 and the light detector 530 located in the through-hole 525. As another example, the side wall 525a of the through-hole 525 may contact the side surface of the light detector 530, and no space may be formed between the side wall 525a and the light detector 530. The plurality of light detectors 530 may detect the light input from the rear surface of the electronic device.

According to an embodiment, the light guide member 520 may not include the side wall 525a, and the light detector 530 and the light guide member 520 may be integrally formed. In this case, the side wall 525a may be replaced by one surface (e.g., a side surface) of the light detector 530, and the one surface of the light detector 530 may include a material that allows total reflection. Furthermore, the first surface 520a and the second surface 520b, which are close to the through-hole 525 may contact one surface of the light detector 530 such that the light is prevented from being emitted to the through-hole 525.

Referring to FIG. 6, the light emitting from the light source 510 may be input to an inside of the light guide member 520 through the light input part 521. The light input to the inside of the light guide member 520 through the light input part 521 may reach the adjacent part 523 that is adjacent to the light detector 530 through the connection part 522. The light that reaches the adjacent part 523 may be emitted to the light guide member 520 and an outside of the electronic device through the plurality of light output parts 524. The lights emitted through the plurality of light output parts 524 may be diffracted or reflected by an external object 610 (e.g., a wrist or a blood vessel of the user) and then are input to the light detector 530. The light detector 530 may detect the light emitted through the plurality of light output parts 524 and diffracted or reflected by the external object 610.

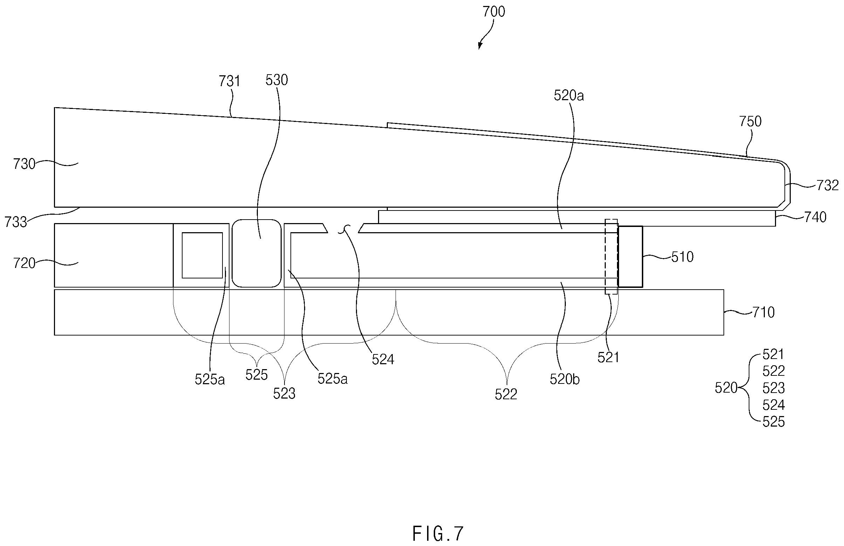

Hereinafter, referring to FIG. 7, an electronic device 700 according to an embodiment will be described. FIG. 7 is a cross-sectional view illustrating the electronic device 700 according to an embodiment.

The electronic device 700 according to an embodiment may include a printed circuit board 710, the light source 510, the light guide member 520, the light detector 530, a magnet member 720, a rear plate 730, a bonding member 740, and an electrode 750 (e.g., the first electrode 231 or the second electrode 232 of FIG. 4).

The printed circuit board 710 may be located inside a housing (e.g., the housing 210 of FIG. 2). Electronic parts, such as a processor, a memory, a communication module, various sensor modules, an interface, or a connection terminal, may be located in the printed circuit board 710. The processor, for example, may include one or more of a central processing unit, an application processor, a graphic processing unit (GPU), a sensor processor, or a communication processor.

The light source 510, the light guide member 520, and the light detector 530 may be located on one surface of the printed circuit board 710. The light guide member 520 may include the light input part 521, the connection part 522, the adjacent part 523, the plurality of light output parts 524, and the through-hole 525.

The light detector 530 may be located in the through-hole 525 of the light guide member 520. The light detector 530 may be surrounded by the side wall 525a of the light guide member 520.

The magnet member 720 may be located on one surface of the light guide member 520, on one surface of the printed circuit board 710. The magnet member 720 may detachably fix the electronic device 700 to an external device (e.g., a charging dock).

The rear plate 730 may be located on the light source 510, the light guide member 520, the light detector 530, and the magnet member 720. The rear plate 730 may constitute at least a portion of the rear surface (e.g., the rear surface 210B of FIG. 3) of the housing. At least a portion of the rear plate 730 may be substantially transparent. The lights emitted from the plurality of light output parts 524 of the light guide member 520 may travel via the rear plate 730 and may be diffracted or reflected by the external object. The light diffracted or reflected by the external object may be input to the light detector 530 inside the electronic device 700 via the rear plate 730.

The bonding member 740 may be located between the rear plate 730 and the light guide member 520 to attach the rear plate 730 and the light guide member 520.

The electrode 750 may be located on at least one surface of the rear plate 730. For example, the electrode 750 may extend from a first surface 731 of the rear plate 730 to a side surface 732 and a second surface 733 that faces the first surface 731. According to an embodiment, the electrode 750 may overlap the light source 510 in a thickness direction thereof.

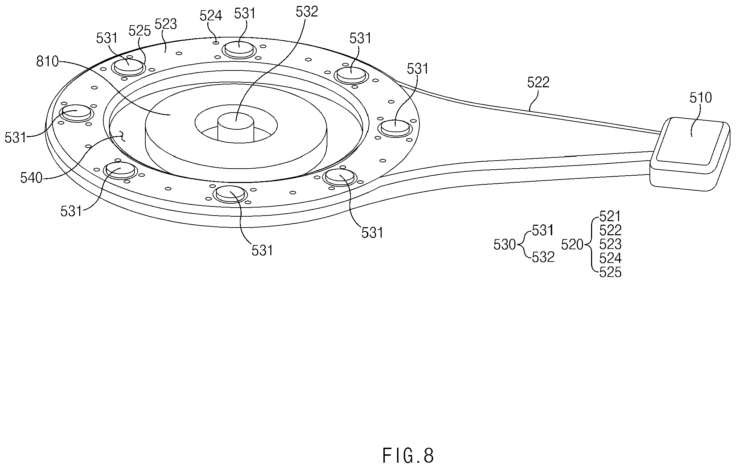

Hereinafter, referring to FIG. 8, some configurations of an electronic device according to an embodiment will be described. FIG. 8 is a view illustrating some configurations of the electronic device according to an embodiment.

The electronic device according to an embodiment may include the light source 510, the light guide member 520, the plurality of light detectors 530, and a magnet member 810. The light guide member 520 may include the light input part 521, the connection part 522, the adjacent part 523, the plurality of light output parts 524, and the plurality of through holes 525. The adjacent part 523 may extend along a circumference of the central area 540.

The plurality of light detectors 530 may include at least one first light detector 531 located in the plurality of through-holes 525 of the light guide member 520, and at least one second light detector 532 located in the central area 540.

The magnet member 810 may be located in the central area 540 while surrounding the second light detector 532. The magnet member 810 may have a ring shape, and the second light detector 532 may be disposed at a central portion of the magnet member 810.

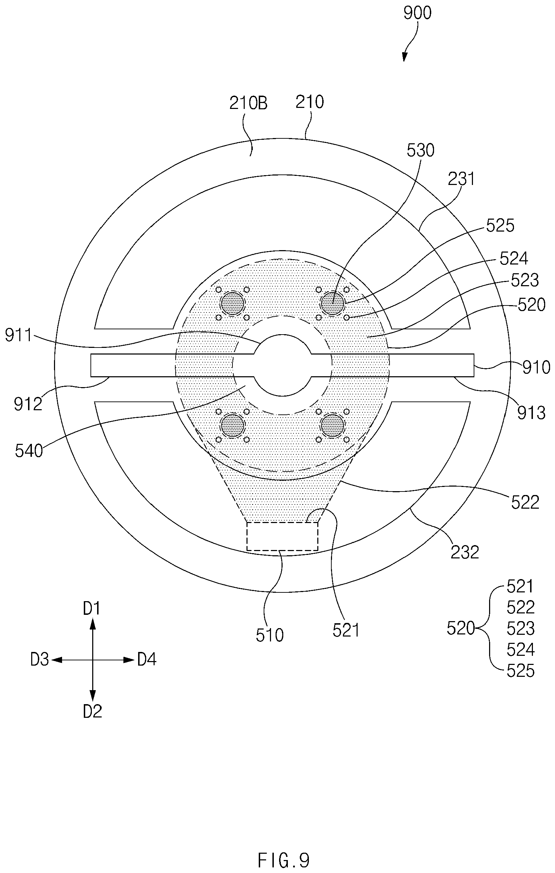

Hereinafter, referring to FIG. 9, some configurations of an electronic device 900 according to an embodiment will be described. FIG. 9 is a rear view of the electronic device 900 according to an embodiment.

The electronic device 900 according to an embodiment may include the housing 210, the light source 510, the light guide member 520, the plurality of light detectors 530, the first electrode 231, the second electrode 232, and a third sensor 910. The light guide member 520 may include the light input part 521, the connection part 522, the adjacent part 523, the plurality of light output parts 524, and the plurality of through-holes 525.

The light source 510 may be located inside the housing 210. For example, the light source 510 may be disposed to overlap at least any one of the first electrode 231, the second electrode 232, and the third sensor 910.

The light input part 521 of the light guide member 520 may be disposed to be adjacent to the light source 510. The adjacent part 523 of the light guide member 520 may extend along a circumference of the central area 540. For example, the central area 540 may be circular, and the adjacent part 523 may have a ring shape. The connection part 522 of the light guide member 520 may extend from the light input part 521 to the adjacent part 523 such that the light input to the light input part 521 reaches the adjacent part 523. The plurality of light output parts 524 may be disposed in a peripheral area of the plurality of through-holes 525, in the adjacent part 523. The plurality of light detectors 530 may be located in the through-holes 525 of the light guide member 520.

The first electrode 231 and the second electrode 232 may be located in the rear surface 210B of the housing 210. The first electrode 231 and the second electrode 232 may be disposed to surround the adjacent part 523 of the light guide member 520. For example, the first electrode 231 and the second electrode 232 may be located along an outskirt of the rear surface 210B of the housing 210. The first electrode 231 may be located on a side of the rear surface 210B of the housing 210 in a first direction D1, and the second electrode 232 may be located on a side of the rear surface 210B of the housing 210 in a second direction D2. The second direction D2 may be an opposite direction to the first direction D1. The adjacent part 523 of the light guide member 520 may be located between the first electrode 231 and the second electrode 232. The first electrode 231 and the second electrode 232 may not overlap the plurality of light output parts 524 and the plurality of light detectors 530. Unlike the illustration, at least a portion of the first electrode 231 and the second electrode 232 may overlap a partial area of the adjacent part 523 of the light guide member 520.

The third sensor 910 may detect third biometric information (e.g., a temperature of skin). For example, the third sensor 910 may be a bimetal temperature sensor, and may include a metal that are different from those of the first electrode 231 and the second electrode 232. The third sensor 910 may be located on the rear surface 210B of the housing 210. At least a portion of the third sensor 910 may be located in the central area 540. The third sensor 910 may include a first sensing part 911 that is located in the central area 540, a second sensing part 912 that extends from the first sensing part 911 in a third direction D3 that crosses the first direction D1, and a third sensing part 913 that extends from the first sensing part 911 in a fourth direction D4 that is an opposite direction to the third direction D3. The second sensing part 912 and the third sensing part 913 of the third sensor 910 may overlap at least a portion of the light guide member 520. The third sensor 910 may not overlap the first electrode 231, the second electrode 232, the plurality of light output parts 524, and the plurality of light detectors 530.

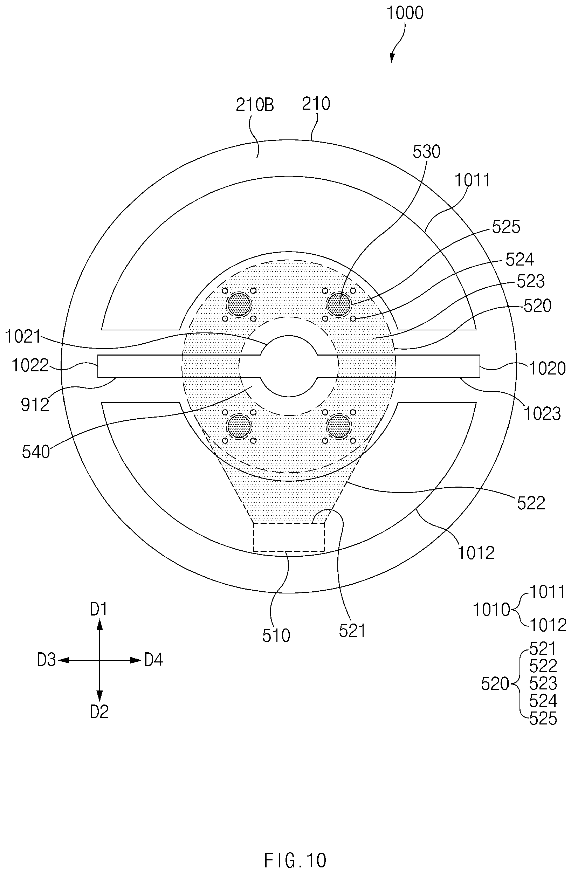

Hereinafter, referring to FIG. 10, some configurations of an electronic device 1000 according to an embodiment will be described. FIG. 10 is a rear view of the electronic device 1000 according to an embodiment.

The electronic device 1000 according to an embodiment may include the housing 210, the light source 510, the light guide member 520, the plurality of light detectors 530, a first electrode 1010, and a second electrode 1020. The light guide member 520 may include the light input part 521, the connection part 522, the adjacent part 523, the plurality of light output parts 524, and the plurality of through-holes 525.

The light input part 521 of the light guide member 520 may be disposed to be adjacent to the light source 510. The adjacent part 523 of the light guide member 520 may extend along a circumference of the central area 540. For example, the central area 540 may be circular, and the adjacent part 523 may have a ring shape. The connection part 522 of the light guide member 520 may extend from the light input part 521 to the adjacent part 523 such that the light input to the light input part 521 reaches the adjacent part 523. The plurality of light output parts 524 may be disposed in a peripheral area of the plurality of through-holes 525, in the adjacent part 523. The plurality of light detectors 530 may be located in the through-holes 525 of the light guide member 520.

The first electrode 1010 and the second electrode 1020 may be located on the rear surface 210B of the housing 210. The first electrode 1010 may correspond to any one of an INP electrode and an RLD electrode, and the second electrode 1020 may correspond to the other. The first electrode 1010 may include a first sub electrode 1011 and a second sub electrode 1012. The first sub electrode 1011 and the second sub electrode 1012 may be disposed to surround the adjacent part 523 of the light guide member 520. For example, the first sub electrode 1011 and the second sub electrode 1012 may be located along an outskirt of the rear surface 210B of the housing 210. The first sub electrode 1011 may be located on a side of the rear surface 210B of the housing 210 in the first direction D1, and the second sub electrode 1012 may be located on a side of the rear surface 210B of the housing 210 in the second direction D2. The first sub electrode 1011 and the second sub electrode 1012 may not overlap the plurality of light output parts 524 and the plurality of light detectors 530. Unlike the illustration, at least a portion of the first sub electrode 1011 and the second sub electrode 1012 may overlap the adjacent part 523 of the light guide member 520.

At least a portion of the second electrode 1020 may be located in the central area 540. The second electrode 1020 may include a first part 1021 that is located in the central area 540, a second part 1022 that extends from the first part 1021 in the third direction D3 that crosses the first direction D1, and a third part 1023 that extends from the first part 1021 in the fourth direction D4 that is an opposite direction to the third direction D3. The second part 1022 and the third part 1023 of the second electrode 1020 may overlap at least a portion of the light guide member 520. The second electrode 1020 may not overlap the plurality of light output parts 524 and the plurality of light detectors 530.

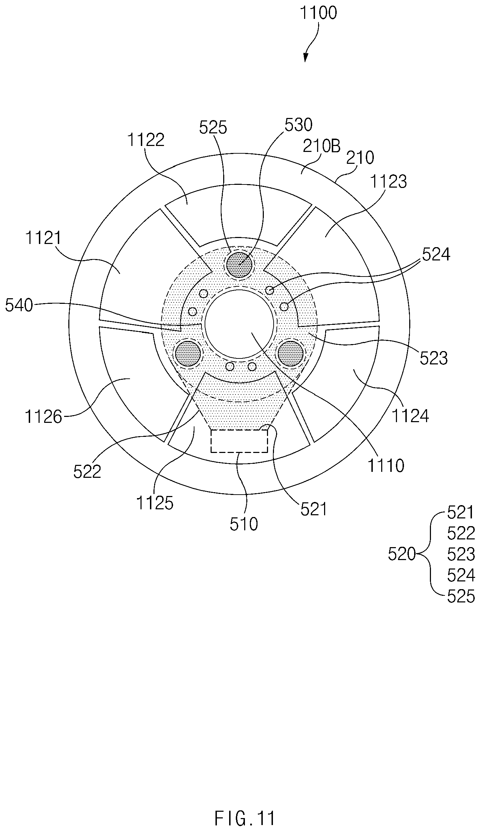

Hereinafter, referring to FIG. 11, some configurations of an electronic device 1100 according to an embodiment will be described. FIG. 11 is a rear view of the electronic device 1100 according to an embodiment.

The electronic device 1100 according to an embodiment may include the housing 210, the light source 510, the light guide member 520, the plurality of light detectors 530, a plurality of electrodes 1121, 1122, 1123, 1124, 1125, and 1126, and a magnet member 1110. The light guide member 520 may include the light input part 521, the connection part 522, the adjacent part 523, the plurality of light output parts 524, and the plurality of through-holes 525.

The light input part 521 of the light guide member 520 may be disposed to be adjacent to the light source 510. The adjacent part 523 of the light guide member 520 may extend along a circumference of the central area 540. For example, the central area 540 may be circular, and the adjacent part 523 may have a ring shape. The connection part 522 of the light guide member 520 may extend from the light input part 521 to the adjacent part 523 such that the light input to the light input part 521 reaches the adjacent part 523. The plurality of light detectors 530 may be located in the through-holes 525 of the light guide member 520. The plurality of light output parts 524 may be arranged along a circumference of the central area 540. Among the plurality of light output parts 524, at least two light output parts 524 may be disposed between any one and another one of the plurality of light detectors 530.

The plurality of electrodes 1121, 1122, 1123, 1124, 1125, and 1126 may be located on the rear surface 210B of the housing 210. The plurality of electrodes 1121, 1122, 1123, 1124, 1125, and 1126 may be disposed to surround the adjacent part 523 of the light guide member 520. For example, the plurality of electrodes 1121, 1122, 1123, 1124, 1125, and 1126 may be located along an outskirt of the rear surface 210B of the housing 210. According to various embodiments, at least one of the plurality of electrodes 1121, 1122, 1123, 1124, 1125, and 1126 may correspond to an INP electrode in the second sensor, and the others may correspond to an RLD electrode. According to an embodiment, the plurality of electrodes 1121, 1122, 1123, 1124, 1125, and 1126 may be configurations that are included in various sensors. For example, at least one of the plurality of electrodes 1121, 1122, 1123, 1124, 1125, and 1126 may be included in an electrocardiogram (ECG), at least another one may be included in a bioelectric impedance analysis (BIA) sensor, at least another one may be included in a galvanic skin response (GSR) sensor, at least another one may be included in an electroencephalogram (EEG) sensor, and the remaining ones may be included in a temperature sensor. The plurality of electrodes 1121, 1122, 1123, 1124, 1125, and 1126 may not overlap the plurality of light output parts 524 and the plurality of light detectors 530. Unlike the illustration, at least some of the plurality of electrodes 1121, 1122, 1123, 1124, 1125, and 1126 may overlap a partial area of the light guide member 520.

The magnet member 1110 may be located in the central area 540. The magnet member 1110 may detachably fix the electronic device 1100 to the external device (e.g., a charging dock).

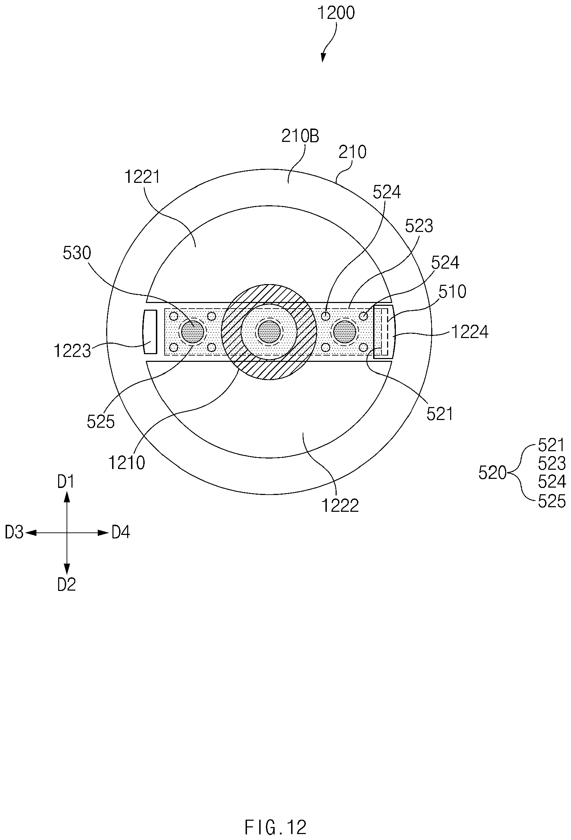

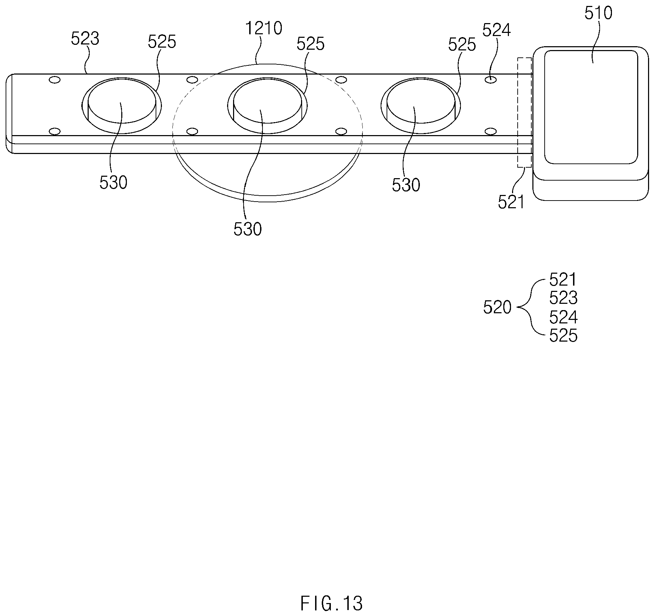

Hereinafter, referring to FIGS. 12 and 13, an electronic device 1200 according to an embodiment will be described. FIG. 12 is a rear view of the electronic device 1200 according to an embodiment. FIG. 13 is a view illustrating some configurations of the electronic device 1200 according to an embodiment.

The electronic device 1200 according to an embodiment may include the housing 210, the light source 510, the light guide member 520, the plurality of light detectors 530, a plurality of electrodes 1221, 1222, 1223, and 1224, and a magnet member 1210. The light guide member 520 may include the light input part 521, the adjacent part 523, the plurality of light output parts 524, and the plurality of through-holes 525.

The light source 510 may be located inside the housing 210. The light source 510 may be disposed to overlap at least any one of the plurality of electrodes 1221, 1222, 1223, and 1224. For example, the light source 510 may be located on a side of the housing 210 in the fourth direction D4.

The light input part 521 of the light guide member 520 may be disposed to be adjacent to the light source 510. The light guide member 520 may extend from one side to an opposite side of the rear surface 210B of the housing 210 in one direction (e.g., the third direction D3). The adjacent part 523 of the light guide member 520 may be disposed to cross the magnet member 1210 on the magnet member 1210. The plurality of light output parts 524 may be disposed in a peripheral area of the plurality of through-holes 525. The plurality of through-holes 525 may be arranged in one direction (e.g., the third direction D3) in the adjacent part 523. At least one of the plurality of through-holes 525 may overlap a center hole 1211 of the magnet member 1210.

The plurality of light detectors 530 may be located in the through-holes 525 of the light guide member 520. The plurality of light detectors 530 may be arranged in one direction (e.g., the third direction D3). At least one of the plurality of light detectors 530 may be located in the center hole 1211 of the magnet member 1210, and may be aligned with the center hole 1211 of the magnet member 1210.

The plurality of electrodes 1221, 1222, 1223, and 1224 may include the first electrode 1221, the second electrode 1222, the third electrode 1223, and the fourth electrode 1224. The first electrode 1221, the second electrode 1222, the third electrode 1223, and the fourth electrode 1224 may be located on the rear surface 210B of the housing 210. The first electrode 1221 may be located on a side of the rear surface 210B of the housing 210 in the first direction D1, and the second electrode 1222 may be located on a side of the rear surface 210B of the housing 210 in the second direction D2. The third electrode 1223 may be located on a side of the rear surface 210B of the housing 210 in the third direction D3, and the fourth electrode 1224 may be located on a side of the rear surface 210B of the housing 210 in the fourth direction D4. At least one of the plurality of electrodes 1221, 1222, 1223, and 1224 may correspond to an INP electrode of the second sensor, and the remaining ones may correspond to an RLD electrode. The plurality of electrodes 1221, 1222, 1223, and 1224 may not overlap the plurality of light output parts 524 and the plurality of light detectors 530. Unlike the illustration, at least some of the plurality of electrodes 1121, 1122, 1123, 1124, 1125, and 1126 may overlap a partial area of the light guide member 520.

The magnet member 1210 may be located at a central portion of the rear surface 210B of the housing 210. The magnet member 1210 may include the center hole 1211, in which the at least one light detector 530 is disposed.

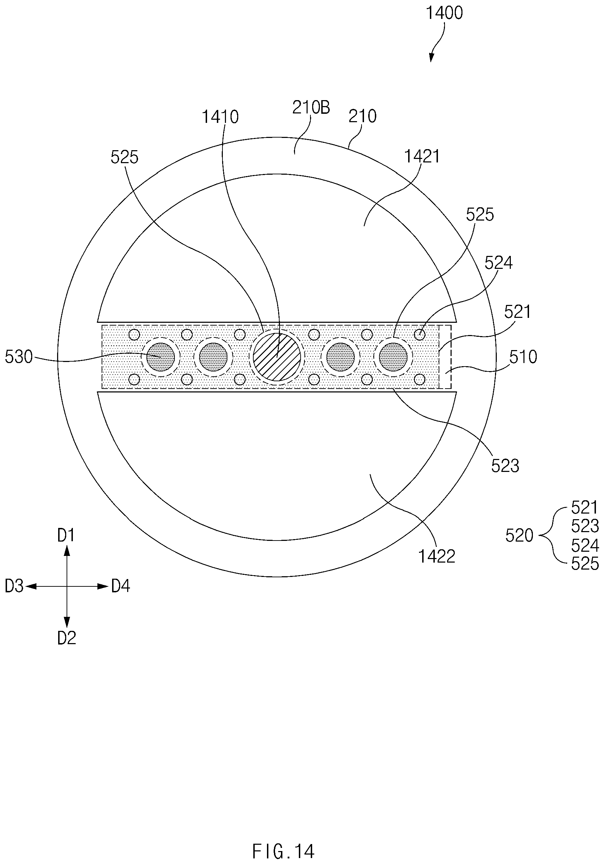

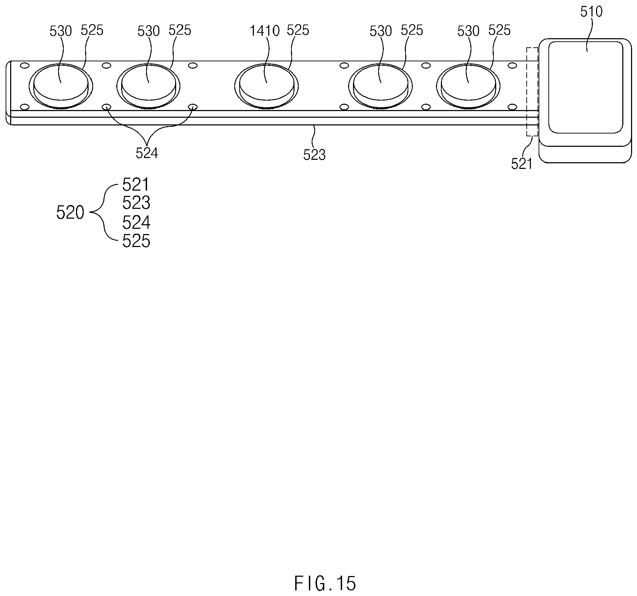

Hereinafter, referring to FIGS. 14 and 15, an electronic device 1400 according to an embodiment will be described. FIG. 14 is a rear view of the electronic device 1400 according to an embodiment. FIG. 15 is a view illustrating some configurations of the electronic device 1400 according to an embodiment.

The electronic device 1400 according to an embodiment may include the housing 210, the light source 510, the light guide member 520, the plurality of light detectors 530, a first electrode 1421, a second electrode 1422, and a magnet member 1410. The light guide member 520 may include the light input part 521, the adjacent part 523, the plurality of light output parts 524, and the plurality of through-holes 525.

The light source 510 may be located inside the housing 210.

The light input part 521 of the light guide member 520 may be disposed to be adjacent to the light source 510. The light guide member 520 may extend from one side to an opposite side of the rear surface 210B of the housing 210 in one direction (e.g., the third direction D3). The plurality of light output parts 524 may be disposed in a peripheral area of the plurality of through-holes 525. The plurality of through-holes 525 may be arranged in one direction (e.g., the third direction D3), in the adjacent part 523.

The plurality of light detectors 530 may be located in the through-holes 525 of the light guide member 520. The plurality of light detectors 530 may be arranged in one direction (e.g., the third direction D3). For example, at least one of the plurality of light detectors 530 may be arranged in the third direction D3 with respect to the magnet member 1410, and the remaining ones may be arranged in the fourth direction D4.

The first electrode 1421 and the second electrode 1422 may be located on the rear surface 210B of the housing 210. The first electrode 1421 may be located on a side of the rear surface 210B of the housing 210 in the first direction D1, and the second electrode 1422 may be located on a side of the rear surface 210B of the housing 210 in the second direction D2. The light source 510, the light guide member 520, the plurality of light detectors 530, and the magnet member 1410 may be located between the first electrode 1421 and the second electrode 1422.

The magnet member 1410 may be located in any one of the plurality of through-holes 525 of the light guide member 520. For example, the magnet member 1410 may be located in the through-hole 525 that is located at a central portion of the rear surface 210B of the housing 210.

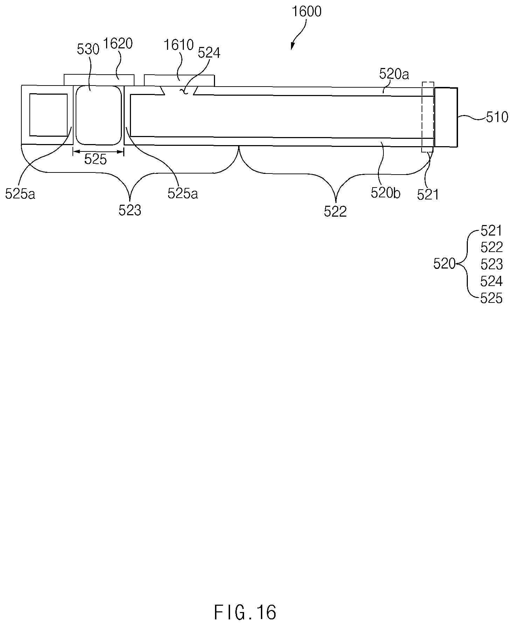

Hereinafter, referring to FIG. 16, a first sensor 1600 (e.g., the first sensor 410 of FIG. 4) included in the electronic device according to an embodiment will be described. FIG. 16 is a cross-sectional view illustrating some configurations included in the electronic device according to an embodiment.

According to an embodiment, the first sensor 1600 may include the light source 510, the light guide member 520, the plurality of light detectors 530, a first optical film 1610, and a second optical film 1620. The light guide member 520 may include the light input part 521, the connection part 522, the adjacent part 523, the plurality of light output parts 524, and the plurality of through-holes 525.

The first optical film 1610 may be located on the plurality of light output parts 524. The first optical film 1610 may change a path of the light such that the lights emitted through the plurality of light output parts 524 diverge. For example, the first optical film 1610 may include a defocusing Fresnel lens.

The second optical film 1620 may be located on the plurality of light detectors 530. The second optical film 1620 may change a path of the light such that the lights input to the plurality of light detectors 530 converge. For example, the second optical film 1620 may include a focusing Fresnel lens.

The first sensor 1600 of the electronic device according to an embodiment includes the first optical film 1610 and the second optical film 1620, and may enhance a biometric information detection performance of the first sensor 1600.

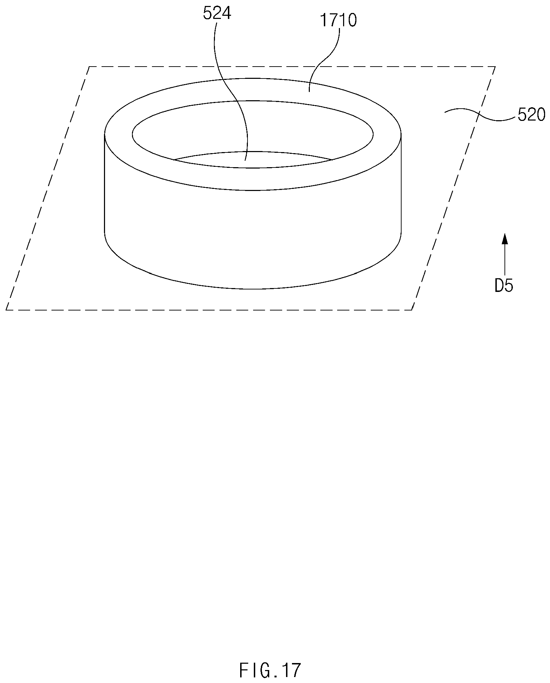

FIG. 17 is a view illustrating some configurations included in an electronic device according to an embodiment.

Referring to FIG. 17, the electronic device according to an embodiment may include the light guide member 520, and a partition wall 1710 that is located in the light output part 524 of the light guide member 520. The partition wall 1710 may protrude along a circumference of the light output part 524. The partition wall 1710 may enhance a straightness of the light by causing the light emitted from an interior of the light guide member 520 through the light output part 524 to travel in a direction (e.g., a fifth direction D5) of the rear surface of the housing.

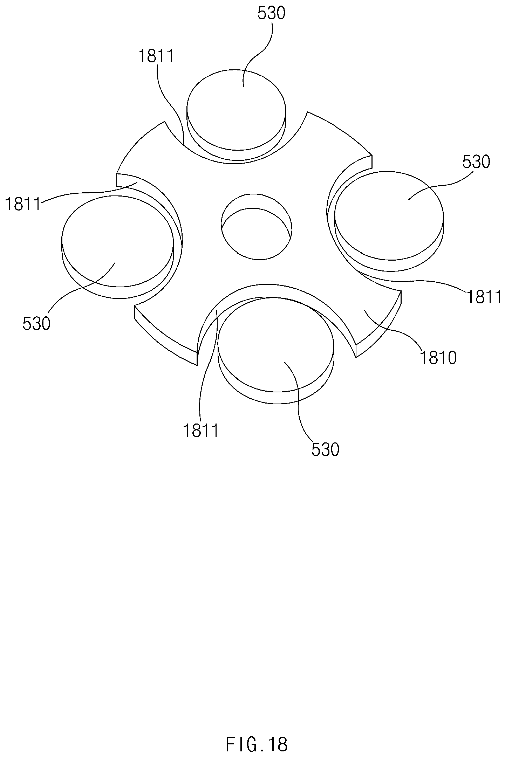

FIG. 18 is a view illustrating some configurations included in an electronic device according to an embodiment.

Referring to FIG. 18, the electronic device according to an embodiment may include a magnet member 1810 and the plurality of light detectors 530. The magnet member 1810 may include a plurality of grooves 1811. For example, the magnet member 1810 may have a "+" shape. The plurality of light detectors 530 may be located in the plurality of grooves 1811 of the magnet member 1810. In the electronic device according to an embodiment, the plurality of light detectors 530 may be located in the plurality of grooves 18111 of the magnet member 1810 to reduce an area occupied by the plurality of light detectors 530 and the magnet member 1810.

A wearable electronic device according to an embodiment may include a housing (e.g., the housing 210 of FIG. 4), and a first sensor (e.g., the first sensor 500 of FIG. 5) that detects first biometric information based on light diffracted or reflected by an external object, the first sensor may include a light source (e.g., the light source 510 of FIG. 5) located inside the housing, a plurality of light detectors (e.g., the light detector 530 of FIG. 5) located inside the housing and that detects the light, and a light guide member (e.g., the light guide member 520 of FIG. 5) extending from an area that is adjacent to the light source to a peripheral area of the plurality of light detectors, and in which the light input to an interior thereof travels while being totally reflected, and the light guide member may include a light input part (e.g., the light input part 521 of FIG. 5) being adjacent to the light source and to which the light emitting from the light source is input, an adjacent part (e.g., the adjacent part 523 of FIG. 5) located at the peripheral area of the plurality of light detectors, and a plurality of light output parts (e.g., the light output part 524 of FIG. 5) located on one surface of the adjacent part and from which the input light is emitted.

In an embodiment, the light guide member may further include at least one through-hole (e.g., the through-hole 525 of FIG. 5) passing from one surface to an opposite surface of the light guide member in the adjacent part, and at least one of the plurality of light detectors may be located in the at least one through-hole.

In an embodiment, the adjacent part may extend along a circumference of a central area of a rear surface of the housing.

In an embodiment, the wearable electronic device may further include a magnet member (e.g., the magnet member 720 of FIG. 7) located in the central area.

In an embodiment, at least one of the plurality of light detectors may be located in the central area.

In an embodiment, the wearable electronic device may further include a magnet member located in the central area, and the magnet member may be disposed to surround the light detector located in the central area.

In an embodiment, the wearable electronic device may further include a second sensor (e.g., the second sensor 230 of FIG. 2) that detects second biometric information, the second sensor may include a first electrode and a second electrode located on the rear surface of the housing, and a third electrode located on another surface of the housing, and the second sensor may be configured to detect the second biometric information based on an electrical signal acquired by using the first electrode, the second electrode, and the third electrode.

In an embodiment, the first electrode may be located on a side of the rear surface of the housing in a first direction, the second electrode may be located on a side of the rear surface of the housing in a second direction, the second direction may be an opposite direction to the first direction, and the first electrode and the second electrode may not overlap the plurality of light detectors and the plurality of light output parts.

In an embodiment, the wearable electronic device may further include a third sensor (e.g., the third sensor 910 of FIG. 9) that detects third biometric information, and the third sensor may include a first sensing part located in the central area, a second sensing part extending from the first sensing part in a third direction, and a third sensing part extending from the first sensing part in a fourth direction that is opposite to the third direction.

In an embodiment, the first electrode may include a first sub electrode located on a side of the rear surface of the housing in a first direction, and a second sub electrode located on a side of the rear surface of the housing in a second direction, and at least a portion of the second electrode may be located in the central area.

In an embodiment, the wearable electronic device may further include a magnet member, and the light guide member may be disposed to cross the magnet member on the magnet member.

In an embodiment, the wearable electronic device may further include a magnet member, and the magnet member may be located in any one of the at least one through-hole.

In an embodiment, the wearable electronic device may further include a first electrode and a second electrode located on the rear surface of the housing, and the light guide member may extend from one side to an opposite side of the rear surface of the housing in one direction, between the first electrode and the second electrode.

In an embodiment, the wearable electronic device may further include a first optical film located on the plurality of light output parts, and that changes a path of the light such that the light emitting through the plurality of light output parts diverges, and a second optical film located on the plurality of light detectors, and that changes a path of the light such that the light input to the plurality of light detectors converges.

In an embodiment, the wearable electronic device may further include a partition wall protruding along a circumference of the plurality of light output parts.

A wearable electronic device according to an embodiment may include a housing (e.g., the housing 210 of FIG. 4), a first sensor (e.g., the first sensor 500 of FIG. 5) that detects first biometric sensor, and a second sensor (e.g., the second sensor 230 of FIG. 2) including a plurality of electrodes located on a rear surface of the housing and that detects second biometric information, the first sensor may include a light source (e.g., the light source 510 of FIG. 5) located on an outside of a central area of a rear surface of the housing, inside the housing, and that overlaps at least any one of the plurality of electrodes, a plurality of light detectors (e.g., the light detector 530 of FIG. 5) located inside the housing and that detects light, and a light guide member (e.g., the light guide member 520 of FIG. 5) extending from an area that is adjacent to the light source to a peripheral area of the plurality of light detectors, and through which the light input to an interior thereof travels while being totally reflected, the light guide member may include a light input part (e.g., the light input part 521 of FIG. 5), to which the light emitted from the light source is input, an adjacent part (e.g., the adjacent part 523 of FIG. 5) located in the peripheral area of the plurality of light detectors, and a plurality of light output parts (e.g., the light output part 524 of FIG. 5) located in the peripheral area of the plurality of light detectors and from which the input light is emitted, and the light output part and the light detector may not overlap the plurality of electrodes.

In an embodiment, the light guide member may further include at least one through-hole that passes from one surface to an opposite surface of the light guide member, in the adjacent part, and at least one of the plurality of light detectors may be located in the at least one through-hole.

In an embodiment, the plurality of electrodes may include a first electrode and a second electrode, and at least a portion of the second electrode may be located in the central area.

In an embodiment, the first electrode may include a first sub electrode located on a side of the rear surface of the housing in a first direction, and a second sub electrode located on a side of the rear surface of the housing in a second direction that is opposite to the first direction, and the second electrode may include a first part located in the central area, a second part extending from the first part in a third direction that crosses the first direction, and a third part extending from the first part in a fourth direction that is opposite to the third direction.

In an embodiment, the light guide member may further include a connection part extending from the light input part to the adjacent part.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable electronic device comprising:
a housing; and
a first sensor configured to detect first biometric information based on light diffracted or reflected by an external object,
wherein the first sensor includes:
a light source located inside the housing;
a plurality of light detectors located inside the housing and configured to detect the light; and
a light guide member extending from an area that is adjacent to the light source to a peripheral area of the plurality of light detectors, and in which the light input to an interior thereof travels while being totally reflected, and
wherein the light guide member includes:
a light input part being adjacent to the light source and to which the light emitting from the light source is input;
an adjacent part located at the peripheral area of the plurality of light detectors; and
a plurality of light output parts located on one surface of the adjacent part and from which the input light is emitted.

2. The wearable electronic device of claim 1, wherein the light guide member further includes at least one through-hole passing from one surface to an opposite surface of the light guide member in the adjacent part, and
wherein at least one of the plurality of light detectors is located in the at least one through-hole.

3. The wearable electronic device of claim 2, wherein the adjacent part extends along a circumference of a central area of a rear surface of the housing.

4. The wearable electronic device of claim 3, further comprising:
a magnet member located in the central area.

5. The wearable electronic device of claim 3, wherein at least one of the plurality of light detectors is located in the central area.

6. The wearable electronic device of claim 5, further comprising:
a magnet member located in the central area,
wherein the magnet member is disposed to surround the light detector located in the central area.

7. The wearable electronic device of claim 3, further comprising:
a second sensor configured to detect second biometric information,
wherein the second sensor includes:
a first electrode and a second electrode located on the rear surface of the housing; and
a third electrode located on another surface of the housing, and
wherein the second sensor is configured to detect the second biometric information based on an electrical signal acquired by using the first electrode, the second electrode, and the third electrode.

8. The wearable electronic device of claim 7, wherein the first electrode is located on a side of the rear surface of the housing in a first direction,
wherein the second electrode is located on a side of the rear surface of the housing in a second direction,
wherein the second direction is an opposite direction to the first direction, and
wherein the first electrode and the second electrode do not overlap the plurality of light detectors and the plurality of light output parts.

9. The wearable electronic device of claim 8, further comprising:
a third sensor configured to detect third biometric information,
wherein the third sensor includes:
a first sensing part located in the central area;
a second sensing part extending from the first sensing part in a third direction; and
a third sensing part extending from the first sensing part in a fourth direction that is opposite to the third direction.

10. The wearable electronic device of claim 7, wherein the first electrode includes:
a first sub electrode located on a side of the rear surface of the housing in a first direction; and
a second sub electrode located on a side of the rear surface of the housing in a second direction, and
wherein at least a portion of the second electrode is located in the central area.

11. The wearable electronic device of claim 2, further comprising:
a magnet member,
wherein the light guide member is disposed to cross the magnet member on the magnet member.

12. The wearable electronic device of claim 2, further comprising:
a magnet member,
wherein the magnet member is located in any one of the at least one through-hole.

13. The wearable electronic device of claim 12, further comprising:
a first electrode and a second electrode located on the rear surface of the housing,
wherein the light guide member extends from one side to an opposite side of the rear surface of the housing in one direction, between the first electrode and the second electrode.

14. The wearable electronic device of claim 1, further comprising:
a first optical film located on the plurality of light output parts, and configured to change a path of the light such that the light emitting through the plurality of light output parts diverges, and
a second optical film located on the plurality of light detectors, and configured to change a path of the light such that the light input to the plurality of light detectors converges.

15. The wearable electronic device of claim 1, further comprising:
a partition wall protruding along a circumference of the plurality of light output parts.
